# EUROPEAN PATENT APPLICATION

(11) **EP 4 732 878 A1**
(43) Date of publication of application: **29.04.2026**
(21) Application number: 25210502.8
(22) Date of filing: 22.10.2025
(51) Int. Cl.: A61M 25/00, A61M 25/01, A61F 2/966, A61F 2/95, B29C 48/09, B29C 48/153, B29C 48/18, B29C 48/21

(54) **SHEATH CATHETER**

(30) Priority: 22.10.2024 US 202463710168 P; 05.12.2024 US 202418970095
(71) Applicant: Micro Medical Solutions, Inc., Woburn, MA 01801 (US)
(72) Inventor: LABRECQUE, Brendan P., Woburn, MA 01801 (US); PAPPAS, Nikolaos D., Woburn, MA 01801 (US)
(74) Representative: Schlich

(57) **Abstract**

A sheath catheter (100) is designed for applicability in intravascular percutaneous procedures, featuring a proximal hub (200), an elongate shaft (300), and a distal radiopaque marker band (400) for enhanced visualization. The elongate shaft (300) incorporates a tri-layer construction: a lubricious polytetrafluoroethylene (PTFE) inner polymer liner to minimize friction during device passage, a braided reinforcement skeleton for superior tensile strength and kink resistance, and a durable, wear-resistant outer jacket to withstand navigational stresses. This configuration yields a low-profile design with exceptional pushability, flexibility, and trackability, enabling access to small, tortuous vessels while maintaining structural integrity.

## Description

### BACKGROUND OF THE INVENTION

### Field of the Invention

The invention relates generally to medical devices, and more particularly to a sheath catheter construction that may form part of a stent delivery device or be an independent catheter construction such as a guiding sheath.

### Description of the Related Art

Within the body, a stenosis or abnormal narrowing may occur in a blood vessel or other tubular organ or structure. In a blood vessel, a stenosis may obstruct flow from the heart to the rest of the body. Within the art, stent devices may be used to treat stenoses. This invention is applicable to the delivery of such devices.

A vascular stent is a small, mesh-like tube that is placed into a stenosed blood vessel to keep it open and restore blood flow. The device acts as a scaffold, supporting the vessel walls and preventing them from collapsing or otherwise causing obstruction. There exist a wide variety of vascular stents used for various applications.

Vascular stents are typically deployed under fluoroscopy using catheters, where the stent is pushed through a tube to its intended location. Such a catheter often includes a sheath catheter and a pusher shaft. Once placed, stents may be either self-expanding or balloon expandable.

The design of the sheath catheter is critical to the performance of a stent deployment catheter. A sheath catheter is subject to a number of often conflicting design requirements such as flexibility, kink resistance, axial tensile and compressive strength, minimized exterior diameter, and maximized interior diameter. In particular, there is a balance between a need for flexibility and a need for strength. Therefore, a need remains for improved medical devices such as stent deployment catheters that are optimized for performance.

### SUMMARY OF THE INVENTION

The invention, as illustrated herein, is clearly not anticipated, rendered obvious, or even present in any of the prior art mechanisms, either alone or in any combination thereof. A novel design and assembly process is disclosed for a sheath catheter used in a stent deployment catheter.

The novel sheath catheter is designed for intravascular percutaneous procedures, featuring a proximal hub, an elongate shaft, and a distal radiopaque marker band for enhanced visualization. The elongate shaft incorporates a tri-layer construction: a lubricious polytetrafluoroethylene (PTFE) inner polymer liner to minimize friction during device passage, a braided reinforcement skeleton tube for superior tensile strength and kink resistance, and a durable, wear-resistant outer jacket. This configuration yields a low-profile design with exceptional pushability, flexibility, and trackability, enabling access to small, tortuous vessels while maintaining structural integrity.

Primarily, the sheath catheter serves as a key component in a stent delivery system, requiring further integration with a stabilizer shaft to facilitate precise, controlled delivery and placement of a preloaded self-expanding stent. The sheath catheter may also be used in ancillary support roles, including as a guiding sheath or microcatheter platform compatible with percutaneous transluminal angioplasty (PTA) balloons, embolic coils, atherectomy devices, angiographic injections, and other interventional tools, thereby streamlining workflows across neurovascular, peripheral, and coronary applications. This multifunctional sheath catheter addresses key challenges in minimally invasive endovascular therapy, offering improved procedural efficiency and patient outcomes.

It is an object of the present system to provide a sheath catheter for use in a variety of catheter applications.

It is a further object of the present invention to provide a sheath catheter with a low wall thickness to allow for easier maneuverability and increased usages during medical procedures.

It is a further object of the present invention to provide a sheath catheter with a low friction interior surface to allow for easier deployment of stents during medical procedures.

It is a further object of the present invention to provide a sheath catheter that maintains superior flexibility, kink resistance, and axial tensile and compressive strength when traversing tortuous paths during medical procedures.

It is a further object of the present invention to provide a sheath catheter with a distal marker band (MB) providing a visual reference under fluoroscopy.

It is a further object of the present invention to provide a sheath catheter with a uniform stiffness, or a customizable stiffness profile along its length to allow for a multitude of clinical applications.

It is a further object of the present invention to provide a sheath catheter with a customizable stiffness profile with a stiff proximal region to allow for increased pushability during medical procedures.

It is a further object of the present invention to provide a sheath catheter with a soft distal region for increased maneuverability during medical procedures.

There has thus been outlined, rather broadly, the more important features of a catheter sheath system, the description thereof that follows may be better understood, and in order that the present contribution to the art may be better appreciated. There are additional features of the system that will be described hereinafter and which will form the subject matter of the claims appended hereto.

In this respect, before explaining at least one embodiment of the system in detail, it is to be understood that the system is not limited in its application to the details of construction, assembly and arrangements of the components set forth in the following description or illustrated in the drawings. The system is capable of other embodiments and of being practiced and carried out in various ways. Also, it is to be understood that the phraseology and terminology employed herein are for the purpose of description and should not be regarded as limiting.

These together with other objects of the system, along with the various features of novelty, which characterize the system, are pointed out with particularity in the claims annexed to and forming a part of this disclosure. For a better understanding of the system, its operating advantages and the specific objects attained by its uses, reference should be made to the accompanying drawings and descriptive matter in which there are illustrated preferred embodiments of the system.

The foregoing has outlined the more pertinent and important features of the present system in order that the detailed description of the system that follows may be better understood, and the present contributions to the art may be more fully appreciated. It is of course not possible to describe every conceivable combination of components and/or methodologies, but one of ordinary skill in the art may recognize that many further combinations or permutations are possible. Accordingly, the novel architecture described below is intended to embrace all such alterations, modifications, and variations that fall within the spirit and scope of the appended claims.

### BRIEF DESCRIPTION OF THE DRAWINGS

Advantages of the present system will be apparent from the following brief description of exemplary embodiments thereof; which description should be considered in conjunction with the accompanying drawings. Having thus described the invention in general terms, reference will now be made to the accompanying drawings, which are not necessarily drawn to scale. The system may be more completely understood in consideration of the following detailed description of various embodiments of the system in connection with the accompanying drawings, in which:
**FIG. 1** is a side cross sectional view of the sheath catheter comprising a proximal hub, elongate shaft, and distal marker band.
**FIG. 2** is a front cross sectional view of the sheath catheter elongate shaft comprising three layers: a lubricious inner polymer liner, a braided skeleton tube, and a wear-resistant outer jacket.
**FIG. 3** is a front cross sectional view of the sheath catheter braided skeleton tube with inner and outer polymer layers.
**FIG. 4** is a perspective view of the sheath catheter braided skeleton tube with metal wires oriented helically and polymer fiber bundles oriented axially.
**FIG. 5** is a side cross sectional view of the sheath catheter proximal hub comprising a Luer fitting insert molded over a strain relief.
**FIG. 6** is a partial cutaway side view of the sheath catheter distal tip with a radiopaque marker band and atraumatic tip.
**FIG. 7** is a flow chart describing the assembly process for the braided skeleton tube.
**FIG. 8** is a flow chart describing the assembly process for the sheath catheter.

While the invention is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the spirit and scope of the invention.

### DETAILED DESCRIPTION OF THE SEVERAL EMBODIMENTS

For the following defined terms, these definitions shall be applied, unless a different definition is given in the claims or elsewhere in this specification. All numeric values are herein assumed to be modified by the term "about", whether or not explicitly indicated. The term "about" generally refers to a range of numbers that one of skill in the art would consider equivalent to the recited value (*i.e.,* having the same function or result). In many instances, the terms "about" may include numbers that are rounded to the nearest significant figure.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following description should be read with reference to the drawings wherein like reference numerals indicate like elements throughout the several views. The drawings, which are not necessarily to scale, depict illustrative embodiments of the claimed invention.

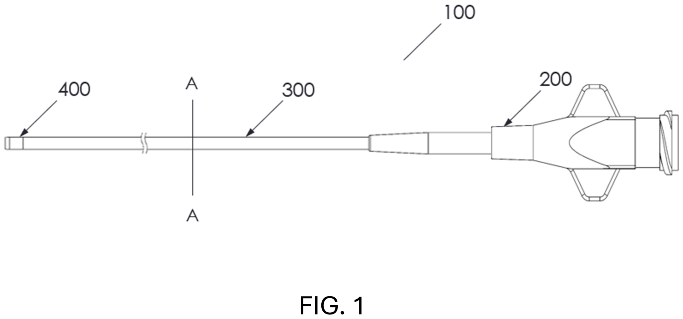

**FIG. 1** illustrates a simple cross-sectional view shown lengthwise of one embodiment of a sheath catheter **100** comprising a proximal hub **200,** elongate shaft **300,** and distal MB **400.** "Proximal" end refers to the end closer to an entry location outside the body. "Distal" end refers to the farthest end from the entry location.

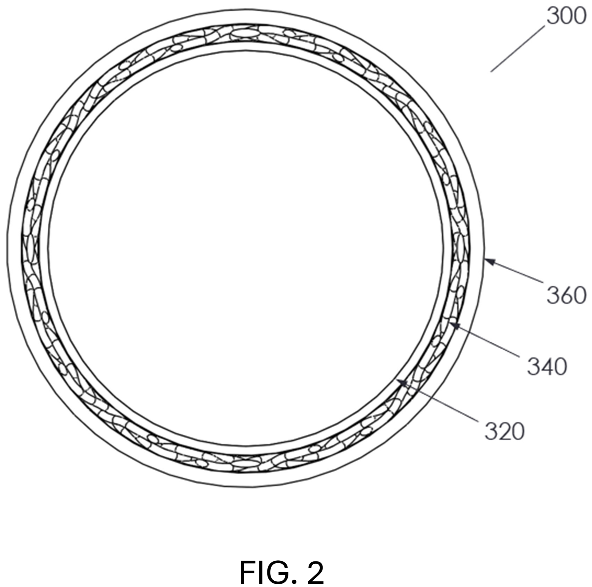

**FIG. 2** illustrates a cross-sectional view along line A-A of the elongate shaft **300** shown in **FIG. 1****,** wherein the elongate shaft **300** comprises three layers: a lubricious inner polymer liner **320,** a braided skeleton tube **340,** and a wear-resistant outer jacket **360.** The lubricious inner polymer liner **320** may be formed of any suitable polymeric material. In various embodiments, the lubricious inner polymer liner **320** may comprise a lubricious polymer such as polytetrafluoroethylene (PTFE), which may be etched to enhance thermal bonding to the braided skeleton tube **340.** In one embodiment, the lubricious inner polymer liner **320** may be of 0.0010" ± 0.0005" thickness, or in another embodiment as thick as 0.002". The wear-resistant outer jacket **360** may be formed of any suitable polymeric material. In various embodiments, the wear-resistant outer jacket **360** may comprise a thermoplastic such as polyamide (*e.g.,* nylon), polyurethane (PU), polyethylene terephthalate (PET), or polyether block amide (PEBA). In one embodiment, the wear-resistant outer jacket **360** may be of 0.002" ± 0.001" thickness, and in another embodiment as thick as 0.005".

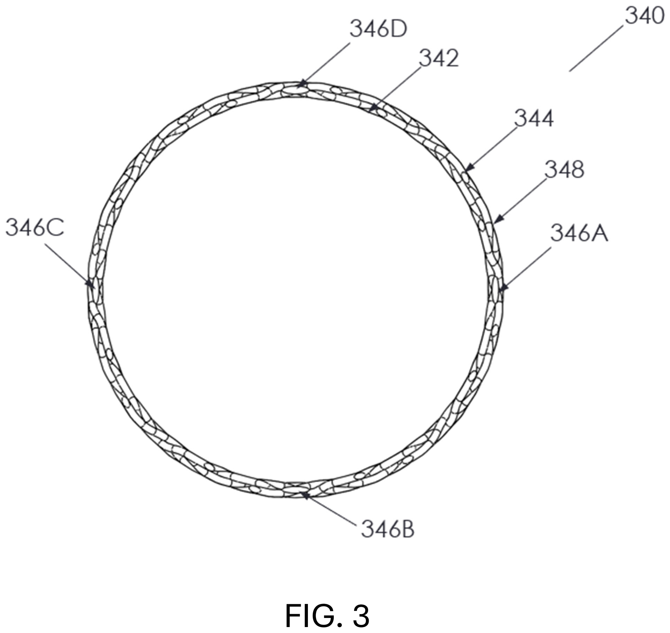

**FIG. 3** provides a detailed view of the braided skeleton tube **340** shown in cross-section in **FIG. 2****,** wherein the braided skeleton tube **340** comprises three layers: an inner polymer layer **342,** a braided body **344,** and an outer polymer layer **348.** In various embodiments, the polymer layers encapsulating the braided skeleton tube comprise a thermoplastic such as polyamide *(e.g.,* nylon), PU, PET, PEBA, or a combination thereof. In various embodiments, the inner polymer layer **342** may be a coating or extrusion applied to continuous malleable copper wire before applying the braided body **344;** and the outer polymer layer **348** may be applied by a coating or extrusion. In one embodiment, the polymer layers **344, 348** encapsulating the braided body **344** are of 0.0005" thickness, and in another embodiment may be 0.00025" thickness or 0.001" thickness.

In various embodiments, the braided body **344** of the braided skeleton tube **340** comprises metal wires oriented helically and polymer fiber bundles oriented axially. In one embodiment, the braided body **344** comprises metal wires **348** of grade 304 or 316 stainless steel (SST) with 0.0005" ± 0.0001" thickness and 0.0025" ± 0.0001" width, braided at 90 ± 40 per inch crosses (PIC) in a 1 wire under - over 2 pattern. In the same embodiment, the braided body **344** further comprises four polymer fiber bundles **346A, 346B, 346C, 346D** of aramid woven in a triaxial pattern at a denier range of 1-200 dtex, spaced equally on the circumference 90° apart.

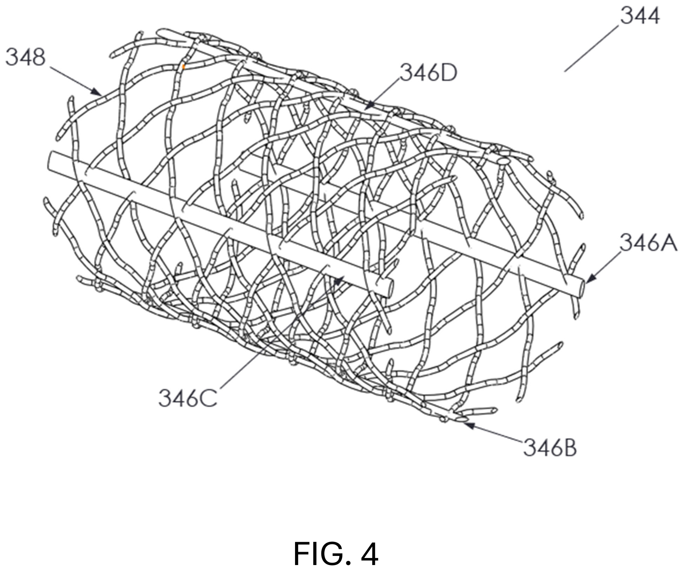

**FIG. 4** shows a perspective view of the same embodiment of the braided body **344,** demonstrating the helical orientation of the metal wires **348** and axial orientation of the four polymer fiber bundles **346A, 346B, 346C, 346D.** In other embodiments, there are one, two, three, five, six, seven, or eight polymer fiber bundles spaced equally on the circumference. In other braided skeleton tube **340** embodiments, the helical braid pattern may be a 1 wire under 2/over 2 pattern or 2 wire under 2/over 2 pattern, and the wire thickness may range from 0.0005" to 0.0050" thickness, and be flat shaped wire or round shaped wire.

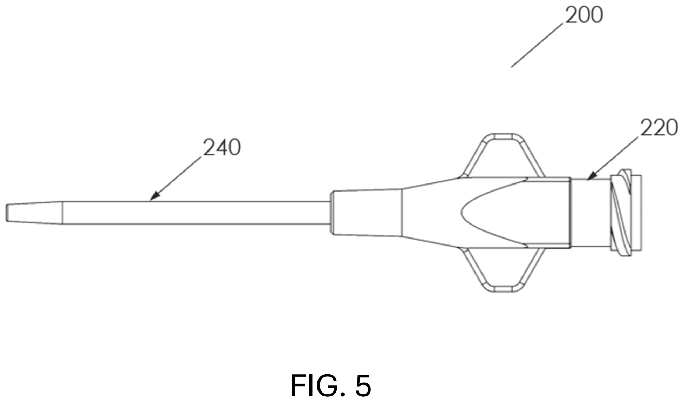

**FIG. 5** illustrates a side cross sectional view of the sheath catheter proximal hub **200** incorporating a Luer fitting **220** insert molded over a strain relief **240.** In various embodiments, the proximal hub **200** comprises a thermoplastic such as polyamide (*e.g.,* nylon), PU, PET, PEBA, or a combination thereof. The Luer fitting **220** provides a means of flushing and deairing the sheath catheter **100.** The strain relief **240** prevents kinking of the elongate shaft **300** during use of the device.

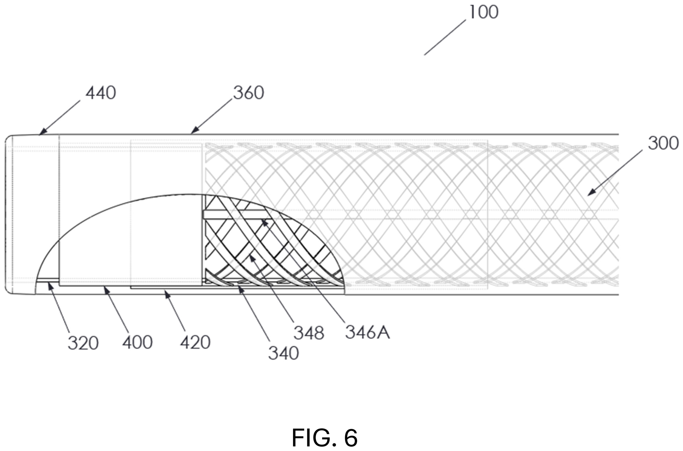

**FIG. 6** illustrates a partial cutaway side view of the sheath catheter **100** distal tip with radiopaque MB **400.** In various embodiments, the MB **400** comprises platinum, platinum-iridium, gold, tantalum, or a tungsten-filled polymer. In one embodiment, the MB **400** is of 0.040" ± 0.005" width and 0.0010" ± 0.0005" thickness. The MB **400** serves as a visual reference under fluoroscopy to enable accurate placement. In one embodiment, a short segment tubular sleeve **420** may be comprised of a polymer having a higher reflow temperature than the skeleton tube outer polymer layer, which constrains the end of the braided skeleton tube **340** during lamination to eliminate braid wire fraying, and one end of the tubular sleeve **420** may partially or completely overlap the marker band **400.** In the same embodiment, the wear-resistant outer jacket **360** is reflowed over the distal half of the MB **400,** segment of the tubular sleeve **420,** and remainder of the elongate shaft **300.** Thus, the MB **400** is fully encapsulated within the atraumatic tip **440** of the sheath catheter **100.**

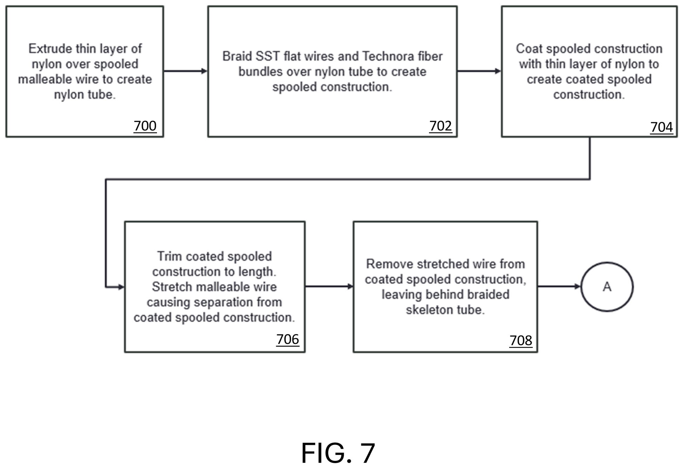

**FIG. 7** illustrates a flow chart describing the braided skeleton tube **340** assembly process, further detail of one embodiment for assembling the braided skeleton tube body is as follows:
1. A spool of continuous malleable copper wire is fed into an extruder, and nylon is extruded over said copper wire, resulting in a thin nylon extrusion, typically 0.0005" thickness **700.**
2. Said spooled nylon tube is fed into a braider where said tube is braided over, typically using a **304** SST flat wire 0.0005" x 0.0025", 90 PIC, with four (4) axial Technora aramid fiber bundles, woven within said flat wires spaced equidistant within the circumference of the tube **702.**
3. Said spooled construction is fed into a coating machine that dispenses a thin layer of nylon, typically 0.0005", encapsulating said nylon tube, braid wires, and axial fiber bundles **704.**
4. Said coated spooled construction is trimmed to length. The malleable copper wire is stretched independently of said construction, reducing the wire's diameter causing separation from said construction **706.**
5. Said stretched copper wire is removed from said coated spooled construction, leaving behind a braided tube construction, termed "braided skeleton tube" **708.**

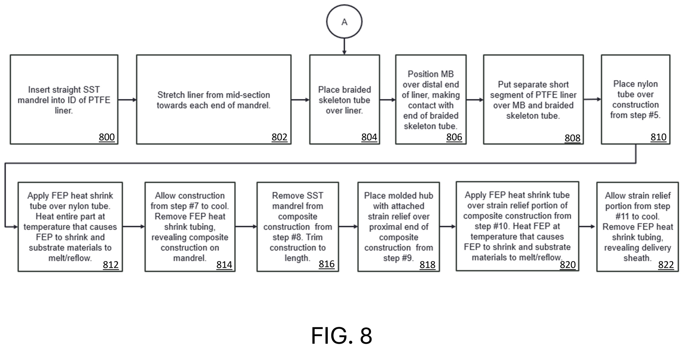

**FIG. 8** illustrates a flow chart describing the sheath catheter assembly process, further detail of one embodiment of assembling the sheath catheter involves the following steps:
1. A long, typically 60", straight SST mandrel with rounded ends, is inserted into the inner diameter (ID) of a PTFE thinwalled lubricious inner polymer liner tube (hereafter "liner") **800.**
2. The mid-section of said liner is stretched axially, reducing the diameter until it contacts the mandrel. The liner is proportionally stretched towards each end of the mandrel. A portion of liner overhanging each end of the mandrel is twisted and/or knotted to prevent liner recoil **802.**
3. The braided skeleton tube is placed over the liner **804.**
4. A MB is placed over the distal end of the liner, making contact with the end of the braided skeleton tube **806.**
5.A separate short segment of liner is placed over said MB and braided skeleton tube, partially covering said parts and bridging the junction **808.**
6.A nylon tube is placed over said construction **810.**
7. FEP heat shrink tubing is placed over the nylon tube and the entire part is heated at a temperature that causes the FEP to shrink and substrate materials to melt/reflow **812.**
8. Said construction is allowed to cool and the FEP heat shrink tubing is removed, revealing a composite construction on the mandrel **814.**
9. The SST mandrel is removed and said composite is trimmed to length **816.**
10. A molded hub with attached strain relief is placed over the proximal end of the composite **818.**
11. FEP heat shrink tubing is placed over the strain relief portion and the FEP is heated at a temperature that causes the FEP to shrink and substrate materials to melt/reflow **820.**
12. Said portion is allowed to cool and the FEP heat shrink removed, revealing said sheath catheter **822.**

## Claims

1. A sheath catheter comprising:
a braided skeleton tube, wherein metal wires are oriented helically in a braided structure with triaxial polymer fiber bundles oriented axially; running longitudinal between the diamonds of the braided wire; and a pair of polymer layers, wherein a first polymer layer encapsulates the inside surface of the braided skeleton tube, and wherein a second polymer layer encapsulates the outside surface of the braided skeleton tube;
a lubricious inner polymer liner, wherein the liner is thermally bonded to the first polymer layer encapsulating the inside surface of the braided skeleton tube;
a polymer wear-resistant outer jacket, wherein the wear-resistant outer jacket is thermally bonded to the second polymer layer encapsulating the outside surface of the braided skeleton tube;
a hub with strain relief thermally bonded to the proximal end of said construction;
a radiopaque marker band thermally bonded to the distal end of said construction;
an atraumatic tip distal of the marker band position of said construction.

2. The sheath catheter of claim 1, wherein the braided skeleton tube comprises a braid of metal wires, of grade 304 or 316 stainless steel, of which the wire thickness ranging between 0.0005" - 0.0050"; and be flat shaped wire or round shaped wire.

3. The sheath catheter of claim 1, wherein the braided skeleton tube reinforcement comprises metal wires braided at 90 ± 40 PIC density in a 1 wire under 1/over 1 pattern, 1 wire under 2/over 2 pattern, or 2 wire under 2/over 2 pattern.

4. The sheath catheter of claim 1, wherein the braided skeleton tube comprises one to eight polymer fiber bundle elements, made of material aramid, with a denier ranging from 1-200 dtex, woven in an axial orientation, and axially spaced equally apart on the circumference.

5. The sheath catheter of claim **1,** wherein the polymer layers encapsulating the braided skeleton tube comprise a thermoplastic such as polyamide *(e.g.,* nylon), PU, PET, PEBA, or a combination thereof; and are of thickness on the interior ranging between 0.00025" thick to 0.0010" thick; and of thickness on the exterior ranging between 0.00025" thick to 0.0010" thick.

6. The sheath catheter of claim **1,** wherein the polymer liner interior to the braided skeleton tube comprises etched PTFE and is of thickness ranging from 0.0005" to 0.0020".

7. The sheath catheter of claim **1,** wherein the polymer wear-resistant outer jacket exterior to the braided skeleton tube comprises a thermoplastic such as polyamide *(e.g.,* nylon), PU, PET, PEBA, or a combination thereof, and is of thickness ranging from 0.001" to 0.005".

8. The sheath catheter of claim **1,** wherein the hub at the proximal end of the braided skeleton tube comprises a Luer fitting and a strain relief, with material composition of a thermoplastic such as polyamide (*e.g.,* nylon), PU, PET, PEBA, or a combination thereof.

9. The sheath catheter of claim **1,** wherein the radiopaque marker band at the distal end of the braided skeleton tube comprises platinum, platinum-iridium, gold, tantalum, or a tungsten-filled polymer.

10. The sheath catheter of claim **1,** wherein the polymer wear-resistant outer jacket has a gradient in durometer to provide a stiff proximal region and a soft distal region; or the polymer wear-resistant outer jacket does not have a gradient in durometer to maintain lower manufacturing burden.

11. The sheath catheter of claim **1,** wherein the inner polymer layer of the braided skeleton tube is formed by coating or extrusion, and wherein the outer polymer layer of the braided skeleton tube is formed by coating or extrusion.

12. The sheath catheter of claim **1,** wherein the distal end of the braided skeleton tube is overlapped by a short segment of tubular sleeve comprised of a polymer having a higher reflow temperature than the skeleton tube outer polymer layer, which constrains the end of the skeleton tube during lamination to eliminate braid wire fraying, and one end of the tubular sleeve may partially or completely overlap the marker band.

13. The sheath catheter of claim **1,** wherein the marker band is encapsulated at the distal end of the braided skeleton tube by reflow of a thermoplastic tubing placed over the length of the delivery shaft forming a polymer wear-resistant outer jacket with an atraumatic tip.

14. The sheath catheter of claim **1,** wherein the proximal hub is formed by a Luer hub fitting insert molded over a strain relief; wherein the strain relief provides a region to thermally attach the Luer hub to the elongate shaft forming a lap shear joint.

15. The sheath catheter of claim 1, wherein the skeleton tube braid layer is assembled to liner without the need to first thermally anneal the metal wires to prevent fraying.
